Europäisches Patentamt

(19) European Patent Office

Office européen des brevets

(11) Numéro de publication : **0 239 468 B1**

(12) # FASCICULE DE BREVET EUROPEEN

(45) Date de publication du fascicule du brevet :
**05.06.91 Bulletin 91/23**

(51) Int. Cl.⁵ : **B08B 3/08, A61L 2/24**

(21) Numéro de dépôt : **87400576.2**

(22) Date de dépôt : **16.03.87**

(54) **Dispositif de régulation de la concentration d'une solution oxydante par mesure du potentiel Redox de cette solution.**

(30) Priorité : **17.03.86 FR 8603745**

(43) Date de publication de la demande :
**30.09.87 Bulletin 87/40**

(45) Mention de la délivrance du brevet :
**05.06.91 Bulletin 91/23**

(84) Etats contractants désignés :
**AT BE CH DE ES GB IT LI NL SE**

(56) Documents cités :
**DE-A- 2 420 327**

(73) Titulaire : **HENKEL FRANCE Société Anonyme
12, avenue Raspail
F-94250 Gentilly Val-de-Marne (FR)**

(72) Inventeur : **Bousser, Charles
5 rue des Prières
Sarry (Marne) (FR)**
Inventeur : **Freal-Saison, Jean-Michel
4 Square des Hortensias
Chatou (Les Yvelines) (FR)**

(74) Mandataire : **Cabinet Pierre HERRBURGER
115, Boulevard Haussmann
F-75008 Paris (FR)**

## Description

La présente invention se rapporte à un dispositif de régulation de la concentration d'une solution oxydante, notamment d'une solution contenant des molécules désinfectantes utilisée à l'échelle industrielle et circulant en circuit fermé dans une canalisation principale faisant, notamment, partie d'une installation de nettoyage et/ou de désinfection par mesure du potentiel redox de cette solution.

L'invention peut, notamment, s'adapter à un système de nettoyage avec récupération de la solution tel que décrit dans le brevet FR-A-2 578 759, ou encore à un système de nettoyage sans récupération de solution, mais comportant un circuit fermé de préparation de cette dernière.

Dans l'industrie alimentaire, et plus particulièrement dans l'industrie laitière, il est en effet nécessaire de faire circuler, après les opérations usuelles de nettoyage et de rinçage, une solution de désinfection oxydante pouvant contenir de l'acide peracétique et/ou de l'eau oxygénée.

Or, pour pouvoir satisfaire aux règles d'hygiène, il est indispensable que cette opération de désinfection permette de détruire au moins tous les micro-organismes pathogènes ou gênants.

Pour obtenir ce résultat, il est nécessaire de contrôler que la concentration en molécules désinfectantes de la solution de désinfection oxydante ne s'abaisse pas au-dessous d'une valeur minima prédéterminée.

Dans ce but, le plus simple est d'effectuer des titrages manuels à intervalles de temps prédéterminés ; de tels titrages sont toujours très longs donc onéreux en main-d'oeuvre : on a, pour cette raison, cherché à les remplacer par des méthodes automatiques.

Parmi les méthodes qui ont été proposées jusqu'à présent, on peut noter l'utilisation d'analyseurs automatiques connus tels que ceux vendus sous la marque "INTEROX" ou "HARSHAW".

Ces appareils permettent un dosage complet de la concentration en molécules oxydantes mais ne peuvent être utilisés industriellement étant donné leur prix d'achat trop élevé, leur maintenance trop onéreuse et leur temps de réponse trop long.

Pour remédier à ces inconvénients, le brevet FR-A-2 578 759 propose un procédé de régulation de la concentration de solutions de désinfection par lequel on mesure, à l'aide d'une sonde de mesure placée dans la solution de désinfection, la valeur d'un paramètre de mesure dépendant de la concentration en molécules désinfectantes, on compare la valeur mesurée à une valeur de consigne, et si la valeur réelle mesurée est différente de la valeur de consigne, on commande le déclenchement d'une pompe doseuse ou l'ouverture d'une électrovanne associée à un réservoir d'une solution concentrée de molécules désinfectantes, de manière à injecter dans la solution de désinfection, des molécules désinfectantes en quantités suffisantes pour rétablir la valeur de consigne de la concentration, cette injection étant automatiquement stoppée lorsque la valeur de consigne est atteinte.

Parmi les paramètres de mesure envisagés, on peut noter la conductivité de la solution désinfectante, son pH, ou encore sa couleur, après réaction chimique caractéristique ou colorimétrique. Ces paramètres possèdent tous leurs avantages et inconvénients propres.

Un autre paramètre dont on peut envisager l'utilisation est le potentiel Redox. Malheureusement, comme on peut s'en rendre compte sur la courbe mentionnée en annexe, qui représente les variations du potentiel (mv) d'une solution en fonction de sa concentration en produits chimiques péroxydés, le potentiel Redox n'est en relation directe avec la concentration que pour de faibles concentrations en molécules désinfectantes, et pour des pH constants.

De plus, lorsque la concentration en molécules désinfectantes croit, la pente de la courbe $rH = f(c)$ décroît très rapidement, jusqu'à ce que cette courbe devienne pratiquement plate donc inexploitable.

En effet, pour des concentrations quelconques, le potentiel Redox d'une solution dépend du pH de celle-ci conformément à la formule :

$$E = Eo + \frac{RT}{nF} \, Ln \, \frac{(Ox)}{(Red)}$$

dans laquelle :

Eo = Potentiel normal d'équilibre,
R = Constante des gaz parfaits,
T = Température absolue,
F = Constante de Faraday,
n = nombre d'électrons mis en jeu.

Cette relation devient, dans le cas des péroxydes, (n = 2) et pour une température de 18°C :
E = 0,029 rH –0,058 pH (rH étant le potentiel Redox).

En conséquence, et comme il a déjà été indiqué dans le brevet FR-A-2 578 759, si l'on veut utiliser en tant que paramètre de mesure le potentiel Redox de la solution désinfectante, il est nécessaire, soit d'effectuer préalablement une dilution de cette dernière, soit de tenir compte du pH de la solution, soit de maintenir le pH constant en ajoutant un élément tampon à la solution désinfectante.

Or, jusqu'à présent, il n'avait jamais été proposé de dispositif permettant la mise en oeuvre, de manière simple, de l'une de ces mesures, et ce, directement sur la solution en circulation.

La présente invention se propose de combler cette lacune en proposant un dispositif de régulation de la concentration d'une solution oxydante, notamment d'une solution contenant des molécules désinfectantes utilisée à l'échelle industrielle et circulant en circuit fermé dans une canalisation principale appartenant, notamment, à une installation dite "centrale de nettoyage en place" ou encore à une installation de préparation en circuit fermé d'une solution de désinfection comportant une cuve de préparation, par mesure du potentiel Redox de cette solution, cette installation comportant une pompe de réajustement ainsi qu'une cellule de mesure comportant une électrode de mesure du potentiel Redox de la solution oxydante et une électrode de mesure du pH de cette solution, associées à un régulateur susceptible de déterminer la valeur réelle en molécules désinfectantes à partir des signaux transmis par la cellule de mesure, de comparer cette valeur réelle à une valeur de consigne, et, si la valeur réelle est différente de la valeur de consigne, de commander la mise en oeuvre de la pompe de réajustement ou de l'électrovanne, de manière à injecter dans la canalisation principale des molécules désinfectantes en quantité suffisante pour rétablir la valeur de consigne de la concentration ; cette injection étant automatiquement stoppée lorsque la valeur de consigne est atteinte, cette installation comportant également un réservoir d'une solution concentrée de molécules désinfectantes, relié à la canalisation principale par une canalisation de réajustement.

Selon l'invention, ce dispositif est caractérisé en ce qu'il comporte une canalisation secondaire branchée en dérivation sur la canalisation principale, en amont de la canalisation de réajustement dans le sens de circulation en circuit fermé de la solution désinfectante, et sur laquelle est montée la cellule de mesure notamment en dérivation ainsi qu'une canalisation d'addition reliée, d'une part à la canalisation secondaire ou à une canalisation annexe montée en dérivation sur cette dernière en amont des électrodes de mesure dans le sens de circulation de la solution désinfectante, et d'autre part, à un réservoir contenant une solution d'adaptation susceptible d'être ajoutée en une proportion déterminée à la solution désinfectante pour abaisser son potentiel Redox à un niveau permettant la mesure et tamponner le pH du milieu, la canalisation secondaire ainsi que la canalisation d'addition et, le cas échéant, la canalisation annexe étant munies d'organes de circulation notamment de pompes, permettant la mise en circulation de la solution désinfectante et de la solution d'adaptation.

Le dispositif conforme à l'invention permet donc de déplacer le point de mesure sur la courbe concentration/potentiel d'oxydoréduction de manière à se trouver dans une zone dans laquelle la variation est rapide, à éviter la polarisation de l'électrode de mesure, et, simultanément, à travailler à pH constant (donc toujours sur la même courbe conformément au schéma figurant en annexe).

L'avantage essentiel de ce dispositif est lié au fait qu'il permet un suivi et un réajustement continu de la concentration en désinfectant.

Après mesure, la solution se trouvant dans la canalisation secondaire est directement conduite à l'égout.

Selon une autre caractéristique de l'invention, le dispositif est muni d'organes de sécurité susceptibles de détecter une anomalie de fonctionnement, notamment la défaillance d'une pompe, l'absence de solution d'adaptation dans la cellule de mesure, etc... et de déclencher une alarme en réponse.

Cet appareillage est également muni dans certains cas de détecteurs de rupture des canalisations de circulation.

Selon une autre caractéristique de l'invention, le régulateur comporte, au moins, un point de consigne associé à la mesure du pH, et comporte, de préférence, deux points de consigne, à savoir un point de consigne haut et un point de consigne bas.

Parmi les régulateurs pouvant être utilisés conformément à l'invention, on peut noter les appareils Dulcometer (marque déposée) disponibles sous les références commerciales RHWS 1000 F1 K2 ; PHWS 014 F1 K2 ; PRW2 F1 K2.

Conformément à cette configuration, le régulateur reçoit donc simultanément les signaux provenant de l'électrode de mesure du potentiel Redox de la solution oxydante qui servent à effectuer la régulation proprement dite et les signaux provenant de l'électrode de mesure du pH qui servent, en réalité, de sécurité.

En effet, si le pH atteint ou dépasse le point de consigne haut, cela signifie qu'il n'y a pas de solution désinfectante ou une concentration anormale en cette dernière au droit des électrodes de mesure ; au contraire, si le pH atteint ou dépasse le point de consigne bas, cela correspond à une absence de réactif d'adaptation ou à une surconcentration anormale de la solution.

Dans les deux cas, le régulateur déclenche une alarme et arrête l'installation.

Selon l'invention, la canalisation secondaire peut n'être pas alimentée en continu, mais comporter une électrovanne montée directement en aval de la canalisation principale ou après une vanne manuelle d'isolement, et alimentant les électrodes de mesure lorsque l'on peut mettre en oeuvre la régulation.

Comme il a déjà été indiqué, le rôle de la solution d'adaptation est de dépolariser l'électrode de mesure du potentiel Redox, de déplacer celui-ci dans une zone de plus forte variation pour augmenter la précision de la régulation et de maintenir le pH constant au moment de la mesure.

A cet effet, et selon une autre caractéristique de l'invention, la solution d'adaptation comporte entre 0 et 50% en poids d'un réducteur, entre 0 et 50% en poids d'un composé alcalin, notamment d'ammoniaque, et entre 0 et 50% en poids d'un élément tampon, notamment d'un sel, par exemple un sel de métal alcalin ou de l'acétate d'ammonium, cette solution étant, le cas échéant, complétée par de l'eau déminéralisée.

Selon l'invention, le réducteur peut être choisi parmi les réducteurs classiques : on peut, par exemple, mentionner les hypophosphites, sulfites, bisulfites ou thiosulfates de métaux alcalins, et les réducteurs organiques tels que l'hydrozine.

Les sels tampons utilisés sont, en fait, similaires aux solutions qui sont utilisées de manière classique pour étalonner les pH mètres.

Bien entendu, les caractéristiques de la solution d'adaptation doivent être avant tout adaptées à la concentration des solutions à réguler ; en effet, le désinfectant peut contenir plus ou moins d'acide selon les opérations industrielles auxquelles il est destiné, ce qui fait qu'il faut un tampon plus ou moins fort pour stabiliser son pH.

La solution d'adaptation est mélangée à la solution à réguler dans un rapport de débit qui varie avec la concentration de la solution désinfectante, de manière à obtenir, généralement, un pH compris entre 5 et 10. Elle peut, en gros, varier de un volume de solution désinfectante pour un volume de solution d'adaptation à un volume de solution d'adaptation pour cinq cents volumes de solution désinfectante.

Selon une autre caractéristique de l'invention, une solution d'adaptation ayant donné particulièrement satisfaction est constituée environ, en pourcentages pondéraux de 2,5% d'ammoniaque, 25% d'acétate d'ammonium, 12,5% de bisulfite de sodium et 60% d'eau déminéralisée.

Selon une autre caractéristique de l'invention, le dispositif comporte un appareil de mesure constituant un ensemble unitaire comportant, d'une part, les pompes de circulation et, d'autre part, une cellule de mesure comportant une chambre de réaction dans laquelle la solution d'adaptation est ajoutée à la solution désinfectante et mélangée à celle-ci, ainsi qu'une chambre de mesure renfermant les deux électrodes de mesure dans laquelle est amenée la solution après mélange.

Les caractéristiques du dispositif qui fait l'objet de l'invention seront décrites plus en détail en se référant aux dessins annexés dans lesquels :
– la figure 1 est un schéma de principe de ce dispositif,
– la figure 2 est une vue en coupe longitudinale de la cellule de mesure,
– la figure 3 est une vue en coupe du couvercle de cette cellule,
– la figure 4 est une vue en coupe d'un piston dont est équipée la chambre de réaction,
– la figure 5 est une coupe de la figure 2, selon l'axe V-V,
– la figure 6 est une coupe de la figure 2, selon l'axe VI-VI.

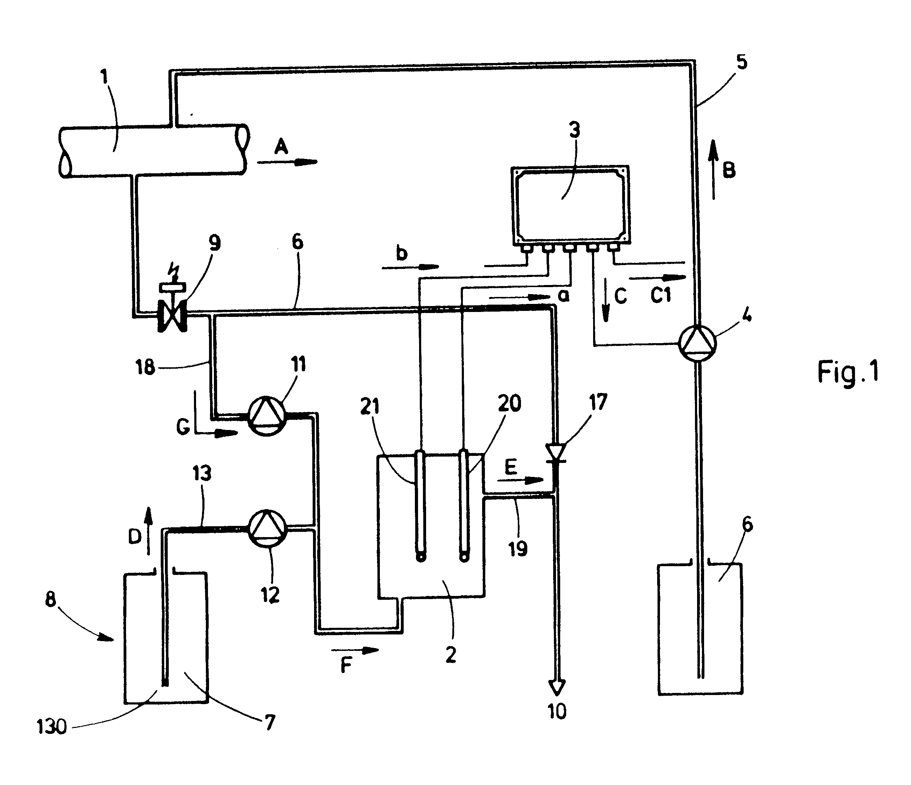

Selon la figure 1, le dispositif objet de l'invention est destiné à réguler la concentration en molécules désinfectantes d'une solution oxydante circulant en circuit fermé dans une canalisation principale 1 faisant partie d'un système dit "centrale de nettoyage en place" ou "circuit court" d'une cuve de préparation de solution, dans le sens schématisé par la flèche A. Cette régulation est mise en oeuvre dans une cellule de mesure 2 comportant une électrode de mesure du potentiel d'oxydoréduction 20 et une électrode de mesure du pH de la solution 21.

Ces électrodes 20, 21 sont associées à un régulateur 3 qui reçoit, conformément aux flèches a et b des signaux transmis par les électrodes 20, 21 et mesure en retour le potentiel Redox qui est en relation directe avec la concentration réelle en molécules désinfectantes de la solution circulant en circuit fermé dans la canalisation 1, conformément à la formule susmentionnée $E = 0,029\ rH - 0,058\ pH$.

Parallèlement à cette mesure, le régulateur 3 compare la concentration réelle en molécules désinfectantes à une valeur de consigne et, dans le cas où la valeur de consigne est différente de la valeur réelle, ce qui signifie que la concentration en molécules désinfectantes de la solution circulant dans la canalisation principale 1 est insuffisante, il envoie, conformément à la flèche c, un signal commandant la mise en oeuvre d'une pompe de réajustement 4 branchée sur une canalisation de réajustement 5 reliée, d'une part, à la canalisation principale 1 et, d'autre part, à un réservoir 6 contenant une solution concentrée de molécules désinfectantes. Donc, un signal c transmis par le régulateur 3 à la pompe de réajustement 4 commande automatiquement l'envoi, selon la flèche B, d'une solution concentrée en molécules désinfectantes dans la canalisation de réajustement 5 jusqu'à obtention de la valeur de consigne de la concentration dans la "centrale de nettoyage en place" à laquelle appartient la canalisation principale 1.

Comme il a déjà été indiqué ci-dessus, le régulateur 3 comporte un point de consigne bas et un point de consigne haut associés à la mesure du pH ; lorsque le signal b transmis par l'électrode 21 de mesure du pH ne correspond pas à un pH compris entre le point de consigne haut et le point de consigne bas, le régulateur 3 émet un signal d'alarme cl qui arrête le dispositif.

Pour permettre de relier dans les meilleures conditions possibles, la mesure du potentiel d'oxydoréduction de la solution oxydante et la concentration réelle en molécules désinfectantes de cette solution, il est utile, non seulement de maintenir le pH constant (c'est-à-dire de se trouver sur une seule et uniquement courbe de pH) mais encore de se trouver dans une zone symbolisée par la lettre X sur les courbes rH = f(c) mentionnées en annexe de façon à avoir une variation rapide du potentiel en fonction de la concentration (zone de forte pente) et à éviter la polarisation de l'électrode 20 de mesure du potentiel d'oxydoréduction.

A cet effet, lors du fonctionnement de la régulation, on ajoute dans la cellule 2 en continu, une proportion déterminée choisie en fonction de la concentration du produit désinfectant d'une solution d'adaptation 7 primitivement contenue dans un réservoir 8.

Pour permettre cet ajout et la mise en oeuvre des différentes mesures, il est prévu une canalisation secondaire 6 branchée sur la canalisation principale 1 en amont de la canalisation de réajustement 5 dans le sens A de circulation de la solution désinfectante. Cette canalisation permet de dériver une partie du liquide de nettoyage circulant dans la "centrale de nettoyage en place" pour la conduire vers la cellule de mesure 2. Cette canalisation 6 qui comporte une électrovanne 9 montée directement ou en aval d'une vanne d'isolement manuelle non représentée sur la figure en aval de la canalisation principale 1, est reliée à l'égout 10 par l'intermédiaire d'un clapet taré 17 dont le rôle est, comme on le verra plus clairement dans la suite de cet exposé, de protéger les électrodes 20 et 21 en évitant toute surpression dans la cellule 2 qui est montée sur une canalisation annexe 18 branchée en dérivation sur la canalisation secondaire 6.

Pour permettre l'introduction au droit des électrodes 20 et 21 de la cellule de mesure 2 d'une proportion prédéterminée de la solution d'adaptation 7 se trouvant dans le récipient 8, ajoutée à la solution désinfectante, il est prévu une canalisation d'addition 13 branchée sur la canalisation annexe 18 en amont de la cellule de mesure 2 dont l'extrémité 130 plonge dans le récipient 8 contenant la solution d'adaptation 7.

La circulation selon la flèche G de la solution désinfectante dans la canalisation annexe 18 et la circulation selon la flèche B de la solution d'adaptation 7 dans la canalisation d'addition 13, sont respectivement commandées par des pompes de circulation 11, 12 placées en amont de la cellule 2. La pompe 11 pourrait également être placée en aval de cette cellule sans pour cela sortir du cadre de l'invention.

Au sortir de la cellule 2, le mélange des solutions provenant des canalisations 18 et 13 est directement transmis à l'égout 10 (flèche E) par une canalisation d'évacuation 19.

En plus des différents éléments susmentionnés, le dispositif pourrait, en outre, comporter d'autres organes non représentés sensibles à une défaillance quelconque du dispositif et susceptibles de déclencher une alarme pour arrêter ce dernier.

Par ailleurs, le dispositif décrit ci-dessus pourrait, conformément à l'invention, être adapté en ne subissant que des modifications mineures non représentées afin de permettre la mise en oeuvre d'opérations de tri entre la solution désinfectante et l'eau de rinçage. Pour pouvoir s'adapter à de tels processus, il serait nécessaire de réduire la distance entre le prélèvement de la solution circulant dans la canalisation principale 1 et les points de mesure (électrodes 20 et 21). Dans ce but, il serait nécessaire de remplacer la pompe 11 du dispositif placée en amont de la cellule de mesure 2 par une pompe placée en aval (ce qui reviendrait à aspirer la solution désinfectante au lieu de la pousser).

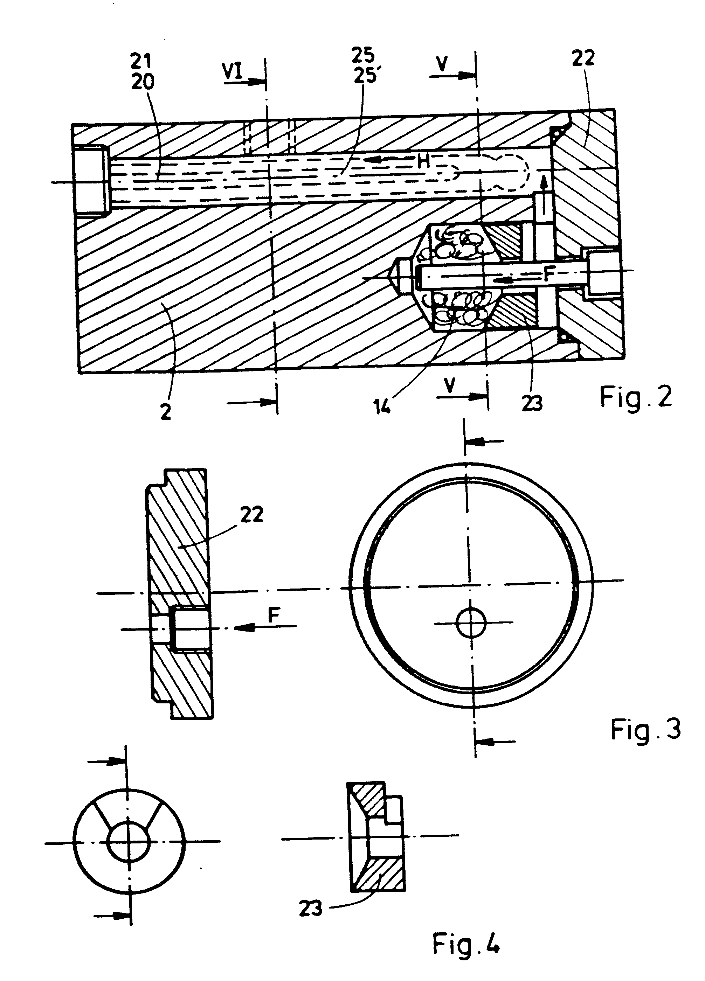

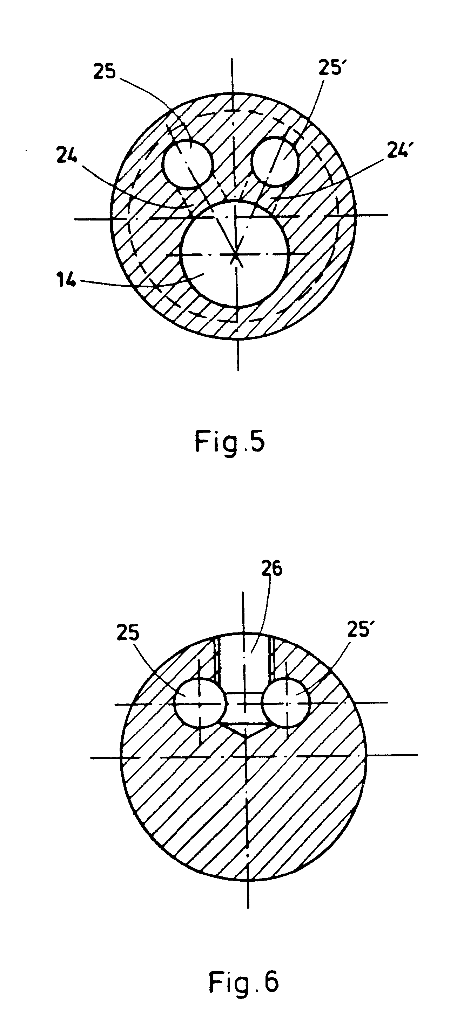

Selon les figures 2 à 6, le mélange solution désinfectante/solution d'adaptation de la canalisation annexe 16, pénètre selon la flèche F dans la cellule de mesure 2 qui est munie d'un couvercle 22. Le mélange à doser pénètre ensuite dans une chambre de réaction 14 dont le volume peut être modifié grâce au piston 23 représenté sur la figure 4. Dans la chambre 14, le liquide à mesurer est soumis à un tourbillonnement vigoureux qui entraîne son mélange avec réaction chimique. Ce mélange peut être amélioré par la présence d'un garnissage du type anneaux de Raschig. Dans un second stade, et comme on peut le voir plus précisément sur la figure 5, le liquide revient en arrière selon la flèche G puis, pénètre dans deux canaux 24, 24' débouchant dans deux chambres de mesure 25, 25' dans lesquelles sont respectivement introduites les électrodes de mesure 20, 21 qui sont représentées en pointillés sur la figure 2.

Après être remonté le long des électrodes 20, 21 selon la flèche H (figure 2) le liquide à mesurer est introduit dans une conduite d'échappement 26 visible sur la figure 6 et pénètre, selon la flèche E, dans la canalisation d'évacuation 19 avant d'être entraîné vers l'égout.

Mesure de potentiel redox sur l'eau oxygénée en
solution dans l'eau dure environ 25° TH

PH 6

PH 7

PH8

mg/l N$_2$O$_2$ à 100 %

mV potentiel redox

300

250

200

150

**Revendications**

1. Dispositif de régulation de la concentration d'une solution oxydante, notamment d'une solution contenant des molécules désinfectantes utilisée à l'échelle industrielle et circulant en circuit fermé dans une canalisation principale (1) faisant notamment partie d'une installation de nettoyage et/ou de désinfection par mesure du potentiel redox de cette solution, dispositif comportant un réservoir (6) d'une solution concentrée de molécules désinfectantes relié à la canalisation principale (1) par une canalisation de réajustement (5) comportant une pompe de réajustement (4) ou tout autre système de distribution tel qu'une électrovanne ainsi qu'une cellule de mesure (2) comportant une électrode (20) de mesure du potentiel Redox de la solution oxydante et une électrode (21) de mesure du pH de cette solution, associées à un régulateur (3) susceptible de déterminer la valeur réelle de la concentration en molécule désinfectantes à partir des signaux transmis par la cellule de mesure, de comparer cette valeur réelle à une valeur de consigne et de commander la mise en oeuvre de la pompe de réajustement (4) ou de l'électrovanne de manière à injecter, dans la canalisation principale (1), des molécules désinfectantes en quantités suffisantes pour rétablir la valeur de consigne de la concentration, cette injection étant automatiquement stoppée lorsque la valeur de consigne est atteinte, dispositif caractérisé en ce qu'il comporte une canalisation secondaire (6) branchée en dérivation sur la canalisation principale (1) en amont de la canalisation de réajustement (5) dans le sens (A) de circulation en circuit fermé de la solution désinfectante, et sur laquelle est montée la cellule de mesure (2), notamment en dérivation, ainsi qu'une canalisation d'addition (13) reliée, d'une part, à la canalisation secondaire (6) ou à une canalisation annexe (18) montée en dérivation sur cette dernière, en amont des électrodes de mesure (20, 21) dans le sens de circulation de la solution désinfectante, et, d'autre part, à un réservoir (8) contenant une solution d'adaptation (7) susceptible d'être ajoutée en une proportion prédéterminée à la solution désinfectante pour abaisser son potentiel Redox à un niveau permettant sa mesure et tamponner le pH du milieu, la canalisation secondaire (6) ainsi que la canalisation d'addition (12) et, le cas échéant, la canalisation annexe (16) étant munie d'organes (9, 11, 12), notamment de pompes, permettant la mise en circulation de la solution désinfectante et de la solution d'adaptation.

2. Dispositif selon la revendication 1, caractérisé en ce que la canalisation secondaire (6) est reliée à l'égout (10).

3. Dispositif selon l'une quelconque des revendications 1 et 2, caractérisé en ce qu'il est muni d'organes de sécurité susceptibles de détecter une anomalie de fonctionnement notamment la défaillance d'une pompe (11, 12, 4), l'absence de solutions d'adaptation (7) dans la cellule de mesure (2), etc.. et de déclencher l'alarme en réponse.

4. Dispositif selon la revendication 3, caractérisé en ce que le régulateur (3) comporte au moins un point de consigne associé à la mesure du pH et de préférence deux points de consigne, à savoir un point de consigne haut et un point de consigne bas.

5. Dispositif selon l'une quelconque des revendications 1 à 4, caractérisé en ce que la canalisation secondaire (6) comporte une électrovanne (9) montée directement en aval de la canalisation principale (20) ou après une vanne manuelle d'isolement.

6. Dispositif selon l'une quelconque des revendications 1 à 5, caractérisé en ce que la solution d'adaptation comporte entre 0 et 50% en poids d'un réducteur, entre 0 et 50% en poids d'un composé alcalin, notamment d'ammoniaque, et entre 0 et 50% en poids d'un élément tampon, notamment d'un sel par exemple un sel de métal alcalin ou de l'acétate d'ammonium, cette solution étant, le cas échéant, complétée par de l'eau déminéralisée.

7. Dispositif selon la revendication 6, caractérisé en ce que le réducteur est choisi dans le groupe formé par les hypophosphites, sulfites, bisulfites ou thiosulfates de métaux alcalins et les réducteurs organiques tels que l'hydrazine.

8. Dispositif selon l'une quelconque des revendications 6 et 7, caractérisé en ce que la solution d'adaptation contient environ, en pourcentages pondéraux, 2,5% d'ammoniaque, 25% d'acétate d'ammoniun, 12,5% de sulfite de sodium et 60% d'eau déminéralisée.

9. Dispositif selon l'une quelconque des revendications 1 à 8, caractérisé en ce qu'il comporte un appareil de mesure constituant un ensemble unitaire comportant, d'une part, les pompes de circulation (11, 12) et, d'autre part, une cellule de mesure (2) comportant une chambre de réaction (14) dans laquelle la solution d'adaptation ajoutée à la solution désinfectante est mélangée à celle-ci ainsi qu'au moins une chambre de mesure (25, 25') renfermant les deux électrodes de mesure (20, 20') dans laquelle est amenée la solution après mélange.

## Ansprüche

1. Vorrichtung zur Regelung der Konzentration einer oxydierenden Lösung, insbesondere einer desinfizierende Moleküle enthaltenden, im industriellen Bereich verwendeten Lösung, die in einem geschlossenen Kreis in einer Hauptleitung (1) zirkuliert, die insbesondere Bestandteil einer Reinigungs- und/oder Desinfektionsanlage ist, durch Messung des Redoxpotentials dieser Lösung, Vorrichtung, enthaltend einen Vorrat (6) einer konzentrierten Lösung desinfizierender Moleküle, der mit der Hauptleitung (1) durch eine eine Einstellpumpe (4) oder eine beliebige andere Verteileinrichtung, wie ein Elektroventil, aufweisende Einstellleitung (5) verbunden ist, sowie eine Meßzelle (2), bestehend aus einer Elektrode (20) zur Messung des Redoxpotentials der oxydierenden Lösung und einer Elektrode (21) zur Messung des pH-Wertes dieser Lösung, die mit einem Regler (3) verbunden ist, der in der Lage ist, aus den von der Meßzelle übermittelten Signalen den tatsächlichen Wert der Konzentration der desinfizierenden Moleküle zu bestimmen, diesen tatsächlichen Wert mit einem Einstellwert zu vergleichen und die Betätigung der Einstellpumpe (4) oder des Elektroventils zu veranlassen, um in die Hauptleitung (1) desinfizierende Moleküle in ausreichender Menge einzubringen, um den Einstellwert der Konzentration wiederherzustellen, wobei dieses Einbringen automatisch beendet wird, wenn der Einstellwert erreicht wird, Vorrichtung, dadurch gekennzeichnet, daß sie eine bezüglich der Richtung (A) der Strömung in geschlossenem Kreis der desinfizierenden Lösung vor der Einstellleitung (5) von der Hauptleitung (1) abgehende Nebenleitung (6) umfaßt, an die die Meßzelle (2), insbesondere in Abzweigung, angeschlossen ist, sowie eine Zufuhrleitung (13), die einerseits an die Nebenleitung (6) oder an eine Abzweigung (18) derselben vor den Meßelektroden (20, 21), bezogen auf die Strömungsrichtung der Desinfektionslösung, angeschlossen ist und andererseits an einen Behälter (8) mit einer Anpassungslösung (7), die geeignet ist, in einem vorbestimmten Verhältnis der desinfizierenden Lösung zugesetzt zu werden, um deren Redoxpotential auf einen dessen Messung ermöglichenden Wert zu senken und den pH-Wert des Mediums zu puffern, wobei die Nebenleitung (6), sowie die Zufuhrleitung (13) und gegebenenfalls die Abzweigung (18) mit Organen (9, 11, 12), insbesondere Pumpen, versehen sind, die es erlauben, das Fließen der desinfizierenden Lösung und der Anpassungslösung zu veranlassen.

2. Vorrichtung gemäß Patentanspruch 1, dadurch gekennzeichnet, daß die Nebenleitung (6) mit der Abwasserleitung (10) verbunden ist.

3. Vorrichtung gemäß irgendeinem der Patentansprüche 1 und 2, dadurch gekennzeichnet, daß sie mit Sicherheitsorganen versehen ist, die geeignet sind, einen Funktionsfehler, insbesondere den Ausfall einer Pumpe (11, 12, 4), das Fehlen von Anpassungslösung (7) in der Meßzelle (2) etc. festzustellen und daraufhin Alarm auszulösen.

4. Vorrichtung gemäß Patentanspruch 3, dadurch gekennzeichnet, daß der Regler (3) mindestens einen mit der Messung des pH-Wertes verbundenen Einstellwert aufweist und vorzugsweise zwei Einstellwerte, nämlich einen hohen und einen niedrigen Einstellwert.

5. Vorrichtung gemäß irgendeinem der Patentansprüche 1 bis 4, dadurch gekennzeichnet, daß die Nebenleitung (6) ein unmittelbar hinter der Hauptleitung (20) oder hinter einen Handabtrennventil angebrachtes Elektroventil (9) aufweist.

6. Vorrichtung gemäß irgendeinem der Patentansprüche 1 bis 5, dadurch gekennzeichnet, daß die Anpassungslösung zwischen 0 und 50 Gewichtsprozent eines Reduktionsmittels, zwischen 0 und 50 Gewichtsprozent einer alkalischen Verbindung, insbesondere einer Ammoniakverbindung, und zwischen 0 und 50 Gewichtsprozent einer Puffersubstanz, insbesondere eines Salzes, beispielsweise eines Alkalimetallsalzes oder von Ammoniumazetat, enthält, wobei diese Lösung gegebenenfalls durch demineralisiertes Wasser vervollständigt wird.

7. Vorrichtung gemäß Patentanspruch 6, dadurch gekennzeichnet, daß das Reduktionsmittel aus der durch die Hypophosphite, Sulfite, Bisulfite oder Thiosulfate von Alkalimetallen und die organischen Reduktionsmittel wie Hydrazin gebildeten Gruppe gewählt wird.

8. Vorrichtung gemäß irgendeinem der Patentansprüche 6 und 7, dadurch gekennzeichnet, daß die Anpassungslösung ungefähr 2,5% Ammoniak, 25% Ammoniumazetat, 12,5% Natriumsulfit und 60% demineralisiertes Wasser, in Gewichtsprozenten, enthält.

9. Vorrichtung gemäß irgendeinem der Patentansprüche 1 bis 8, dadurch gekennzeichnet, däß sie einen eine Einheit bildenden Meßapparat enthält, bestehend aus einerseits den Zirkulationspumpen (11, 12) und andererseits einer Meßzelle (2), die eine Reaktionskammer (14) aufweist, in der die der desinfizierenden Lösung zugesetzte Anpassungslösung mit dieser gemischt wird, sowie mindestens eine Meßkammer (25, 25'), die die beiden Meßelektroden (20, 20') enthält und in die die Lösung nach der Mischung gebracht wird.

**Claims**

1. A device for regulating the concentration of an oxidizing solution, in particular a solution containing disinfectant molecules employed on an industrial scale and circulating in a closed cycle in a main pipeline (1) which in particular forms part of a cleaning and/or disinfecting installation, as a measure of the redox potential of said solution, which device comprises a vessel (6) containing a concentrated solution of disinfectant molecules and connected to the main pipeline (1) via an adjusting pipeline (5) comprising an adjusting pump (4) or any other distribution system, such as a solenoid valve, and a measurement cell (2) comprising an electrode (20) for measuring the redox potential of the oxidizing solution and an electrode (21) for measuring the pH of this solution, connected to a regulating device (3) capable of determining the actual value of the concentration of disinfectant molecules on the basis of signals transmitted by the measurement cell, comparing said actual value with a control value, and actuating the adjustment pump (4) or the solenoid valve so as to inject disinfectant molecules into the main pipeline (1) in sufficient quantities to re-establish the control value of the concentration, said injection being automatically stopped when the control value has been reached, which device is characterised in that it comprises a secondary pipeline (2) connected in parallel with the main pipeline (1) upstream of the readjustment pipeline (5) in the direction (A) of the closed cycle circulation of the disinfectant solution, and on which the measurement cell (2) is mounted, in particular in parallel, as well as an additional pipeline (13) connected on the one hand to the secondary pipeline (6) or to a supplementary pipeline (18) mounted in parallel with the latter, upstream of the measuring electrodes (20, 21) in the direction of circulation of the disinfectant solution, and on the other hand to a vessel (8) which contains an adaptive solution (7) which can be added in a predetermined proportion to the disinfectant solution in order to reduce its redox potential to a level which allows its measurement and to buffer the pH of the medium, the secondary pipeline (6) and the additional pipeline (12) and, where appropriate, the supplementary pipeline (18) being equipped with components (9, 11, 12), in particular pumps, which allow the disinfectant solution and the adaptive solution to be circulated.

2. A device as claimed in claim 1, characterised in that the secondary pipeline (6) is connected to the drain (10).

3. A device as claimed in any one of claims 1 and 2, characterised in that it is equipped with security components capable of detecting a functioning anomaly, in particular the malfunctioning of a pump (11, 12, 4), the absence of adaptive solutions (7) in the measurement cell (2), etc, and of triggering the alarm in response.

4. A device as claimed in claim 3, characterised in that the regulating device (3) comprises at least one control point associated with the measurement of the pH, and preferably two control points, i.e. a high control point and a low control point.

5. A device as claimed in any one of claims 1 to 4, characterised in that the secondary pipeline (6) comprises a solenoid valve (9) mounted directly downstream of the main pipeline (20) or following a manual isolating valve.

6. A device as claimed in any one of claims 1 to 5, characterised in that the adaptive solution comprises between 0 and 50% by weight of a reducing agent, between 0 and 50% by weight of an alkaline compound, in particular ammonia, and between 0 and 50% by weight of a buffer element, in particular a salt, for example a salt of an alkaline metal or of ammonium acetate, said solution being topped up, where necessary, by demineralised water.

7. A device as claimed in claim 6, characterised in that the reducing agent is selected from the group comprising hypophosphites, sulphites, bisulphites or thiosulphates of alkaline metals, and organic reducing agents such as hydrazine.

8. A device as claimed in any one of claims 6 and 7, characterised in that the adaptive solution contains, by weight %, approximately 2.5% ammonia, 25% ammonium acetate, 12.5% sodium sulphite and 60% demineralised water.

9. A device as claimed in any one of claims 1 to 8, characterised in that it comprises a measuring apparatus which constitutes a unified assembly comprising on the one hand circulating pumps (11, 12) and on the other hand a measuring cell (2) which comprises a reaction chamber (14) in which the adaptive solution, added to the disinfectant solution, is mixed with the latter, and at least one measuring chamber (25, 25′) which houses the two measuring electrodes (20, 20′) and to which the solution is conducted after the mixing.

Fig.1

Fig. 2

Fig. 3

Fig. 4

Fig.5

Fig.6